# EUROPÄISCHE PATENTSCHRIFT

(11) **EP 1 339 341 B1**
(45) Veröffentlichungstag und Bekanntmachung des Hinweises auf die Patenterteilung: **01.03.2006**
(21) Anmeldenummer: 01995544.2
(22) Anmeldetag: 29.11.2001
(51) Int. Cl.: A61B 19/00, A61B 1/06

(54) **STIRN-LEUCHTEN/KAMERA-EINHEIT INSBESONDERE FÜR MEDIZINISCHE ANWENDUNGEN**
HEADLAMP/CAMERA UNIT, ESPECIALLY FOR MEDICAL USES
UNITE FRONTALE LAMPE/CAMERA, DESTINEE NOTAMMENT A DES APPLICATIONS MEDICALES

(30) Priorität: 29.11.2000 DE 10059228; 30.11.2000 DE 10059724; 02.06.2001 DE 10126907
(43) Veröffentlichungstag der Anmeldung: 03.09.2003
(73) Patentinhaber: Henning, Wolfram, 90584 Allersberg (DE); Melder, Karl, 74722 Buchen (DE); Schure, Frank, 74722 Buchen (DE)
(72) Erfinder: Henning, Wolfram, 90584 Allersberg (DE); Melder, Karl, 74722 Buchen (DE); Schure, Frank, 74722 Buchen (DE)
(74) Vertreter: Blaumeier, Jörg
(86) Internationale Anmeldenummer: PCT/DE2001/004469
(87) Internationale Veröffentlichungsnummer: WO 2002/044611

(56) Entgegenhaltungen:
- DE-A- 19 542 835
- US-A- 4 616 257
- US-A- 4 797 736
- US-B1- 6 224 227

## Beschreibung

### Technisches Gebiet

Die Erfindung bezieht sich Stirn-Leuchten/Kamera-Einheit insbesondere für medizinische Anwendungen.

### Stand der Technik

Auf einer Vielzahl von Gebieten werden Stirnleuchten eingesetzt, die beispielsweise an einem Stirnband oder an einer Kopfhaube bzw. einem Helm getragen werden. Nur beispielhaft sollen der Bergbau sowie medizinische und insbesondere chirurgische Anwendungen genannt werden. Weiterhin finden derartige Stirnleuchten bei der Montage Anwendung. Die vorstehende Aufzählung ist selbstverständlich nicht vollständig.

Stirnleuchten haben den Vorteil, dass sie den Bereich beleuchten, den die Person, der sie trägt, betrachtet, ohne dass stationäre Scheinwerfer oder in der Hand gehaltene Lampen benötigt würden.

Andererseits werden in der Medizintechnik - aber auch in anderen Bereichen - seit einiger Zeit verstärkt Dokumentationssysteme eingesetzt. Der Einsatz derartiger Systeme dient zum einen der Dokumentation im Falle eventueller Haftungsfälle und zum anderen der ISO-Zertifizierung. Bei Operationen werden im Rahmen derartiger Dokumentationssysteme in der Regel Videokameras verwendet, mit denen der Operationsvorgang aufgenommen und mittels eines Recorders aufgezeichnet wird. Gemäß dem Stand der Technik sind diese Videokameras beispielsweise im Zentrum der Operationsleuchte oder an einer anderen Stelle über dem Operationstisch angeordnet. Nachteilig bei dieser Anordnung der Videokamera ist, dass das eigentliche Operationsfeld oftmals durch den operierenden Arzt oder diesem assistierende Personen verdeckt wird.

Wenn die Videokamera nicht an oder im Zentrum der Operationsleuchte angeordnet ist, ergeben sich darüber hinaus Probleme durch Schatten im Bildfeld, die vom O-perationspersonal etc. erzeugt werden.

Ähnliche Probleme ergeben sich auch bei anderen Einsatzfällen, bei denen stationäre Videokameras verwendet werden.

Andererseits ist der Einsatz beweglicher Videokameras bedienungsaufwendig, und erfordert in der Regel eine Bedienungsperson, die sich nur auf die Videoaufnahme konzentriert.

### Darstellung der Erfindung

Der Erfindung liegt die Aufgabe zugrunde, eine Anordnung für eine Videokamera anzugeben, die eine Verdeckung des zu bearbeitenden Bereichs und insbesondere des Operationsfeldes sowie eine Schattenbildung und/oder die Aufnahme eines anderen Gebietes als die gewünschte Stelle in nahezu allen Einsatzfällen ausschließt.

Weiterhin sollen Stirn-Leuchten/Kamera-Einheiten angegeben werden, die eine Direktübertragung des aufgenommenen Bildes und die Kommunikation mit anderen Arbeitsplätzen ermöglichen.

Ferner sollen bei einer weiteren Ausgestaltung der Erfindung Aufnahmen aus mehreren Perspektiven und insbesondere Stereoaufnahmen möglich sein.

Erfindungsgemäße Lösungen sind in den unabhängigen Patentansprüchen angegeben. Weiterbildungen der Erfindung sind Gegenstand der abhängigen Ansprüche.

Erfindungsgemäß ist erkannt worden, dass eine Verdeckung des zu bearbeitenden Bereichs und insbesondere des Operationsfeldes bei medizinischen Anwendungen dadurch mit hoher Wahrscheinlichkeit ausgeschlossen werden kann, dass die Kamera, die insbesondere eine elektronische Kamera, wie eine Videokamera sein kann, am Kopf der die jeweilige Tätigkeit ausführenden Person, also beispielsweise des operierenden Arztes angeordnet wird. Diese Person wird bereits aufgrund der Tatsache, dass sie ein freies Sichtfeld haben will, darauf achten, dass sich keine Gegenstände oder andere Personen zwischen ihrem Kopf und dem von ihr zu bearbeitenden Bereich, also beispielsweise dem Operationsfeld befinden.

Erfindungsgemäß wird deshalb eine Stirn-Leuchten/ Kamera-Einheit geschaffen, die wenigstens ein Leuchtmittel und eine insbesondere elektronische Kamera aufweist, die an einer Halteeinrichtung angebracht sind, mittels derer das wenigstens eine Leuchtmittel und die Kamera am Kopf einer Person gehalten werden. Die Halteeinrichtung kann dabei beispielsweise ein Stirnband oder eine Kopfhaube bzw. einen Helm aufweisen.

Erfindungsgemäß ist weiterhin erkannt worden, dass auch bei einer Anordnung der Kamera am Kopf und insbesondere an der Stirn einer Bedienungsperson nicht unbedingt gewährleistet ist, dass sich die primär interessierende Stelle im Zentrum des Bildfeldes befindet. Deshalb ist erfindungsgemäß eine optische Zieleinrichtung vorgesehen, die wenigstens eine Marke in das von dem (den) Leuchtmittel(n) beleuchtete Bildfeld der Kamera projiziert.

Damit kann die Bedienungsperson, die die Kamera an ihrem Kopf trägt, zu jedem Zeitpunkt überprüfen, ob sich der interessierende Bereich im Zentrum des von der Kamera aufgenommen Bereichs befindet. Sollte im Einzelfalle beabsichtigt sein, dass sich dieser Bereich außerhalb des Zentrums befindet, oder sollte zu Dokumentationszwecken in bestimmten Sequenzen das Umfeld aufgenommen werden, so kann dies die Bedienungsperson durch eine entsprechende Kopfbewegung leicht ausführen.

Anders ausgedrückt ermöglicht es die Zieleinrichtung der Bedienungsperson, die die Kamera trägt, gezielt bestimmte Szenen aufzunehmen, ohne dass die Bedienungsperson hierzu einen Monitor oder dgl. beobachten müßte.

Für eine besonders effektive Ausleuchtung des Bildfeldes ist es besonders bevorzugt, wenn die Lichtaustrittsfläche(n) des bzw. der Leuchtmittel das Objektiv der Kamera umgibt (umgeben).

Für die Ausbildung der Zieleinrichtung gibt es die verschiedensten Möglichkeiten:

Beispielsweise kann die Zieleinrichtung die Marke in das Zentrum des von der Kamera aufgenommen Bildfeldes projizieren. Die Bedienungsperson weiß damit zu jedem Zeitpunkt, welche Stelle sich im Zentrum des aufgenommenen Bildes befindet. Diese Ausbildung hat den Vorteil, dass lediglich eine Marke projiziert werden muß.

Alternativ oder zusätzlich kann die optische Zieleinrichtung mehrere Marken projizieren:

So ist es möglich, dass die Marken die Begrenzung des Bildfeldes angeben. Diese Ausbildung hat den Vorteil, dass die Bedienungsperson jederzeit über die Begrenzung des aufgenommenen Bereichs informiert ist.

Bei einer weiteren Ausgestaltung der Erfindung vereinigen sich die Marken dann in einem Punkt, wenn der Ort, auf dem sie auftreffen, im Fokusbereich der Kamera liegt. Diese Ausbildung hat den Vorteil, das die Bedienungsperson insbesondere dann, wenn die Kamera über keinen Autofokus verfügt, immer darüber informiert ist, ob sich der aufgenommene Bereich im Schärfenbereich der Kamera befindet.

Für die Ausbildung der optischen Zieleinrichtung sind die verschiedensten Ausgestaltungen möglich, beispielsweise kann die optische Zieleinrichtung wenigstens einen Laserpointer aufweisen.

Sollten die im wesentlichen der Orientierung der Bedienungsperson dienenden Marken bei der Betrachtung des aufgenommenen Bildes stören, so können die Marken im Bild der Kameraeinheit elektronisch oder optisch ausgeblendet werden.

Als Beleuchtungseinrichtungen können Einrichtungen ähnlich den Einrichtungen verwendet werden, wie sie beispielsweise in der Endoskopie eingesetzt werden. Selbstverständlich ist es beim Einsatz von Lampen hoher Leistung möglich, IR-Sperrfilter etc. zu verwenden:

Bei einer bevorzugten Ausgestaltung der Erfindung weist die Beleuchtungseinrichtung wenigstens eine Lichtleitfaser auf, in die das Beleuchtungslicht eingekoppelt wird, und an deren Lichtaustrittsende eine Linse angeordnet ist, die den austretenden Lichtkegel auf das Bildfeld abbildet. Besonders bevorzugt ist es dabei, wenn eine Vielzahl von Lichtleitfasern vorgesehen und jeder Lichtleitfaser eine Linse derart zugeordnet ist, dass sich die Lichtbündel der einzelnen Lichtleitfasern im Bildfeld der Kamera überlagern.

Dabei ist es von besonderem Vorteil, wenn jede Lichtleitfaser einen Durchmesser von ca. 0,5 mm hat. Das aus derartigen Fasern austretende Licht kann mit kleinen Linsen, die nahe an dem Lichtaustrittsende der Lichtleitfaser angeordnet sind, bei üblichen Arbeitsabständen von 60 bis 80 Zentimetern leicht auf einen Durchmesser von 100 mm und mehr aufgeweitet werden. Die Linsen können dabei sogar Teil des Lichtaustrittsendes sein und durch geeignetes Aufschmelzen des Endes hergestellt werden.

Verwendet man z. B. ca. 100 Fasern, so können deren Lichtaustrittsenden leicht ringförmig um das Objektiv der Kamera herum angeordnet werden, und das Licht einer Beleuchtungseinrichtung mit mehreren 100 Watt auf das Bildfeld der Kamera leiten. Die Beleuchtungseinrichtung weist hierzu typischerweise wenigstens eine Lampe und insbesondere eine Gasentladungslampe auf, deren Licht in die Lichtleitfaser(n) eingekoppelt wird.

Alternativ kann die Beleuchtungseinrichtung wenigstens eine Leuchtdiode aufweisen. Dabei kann die wenigstens eine Leuchtdiode distal angeordnet sein, und unmittelbar das bzw. die Leuchtmittel bilden. Als Leuchtdioden können Weißlichtdioden oder im UV- oder IR-Bereich strahlende Dioden eingesetzt werden. Natürlich können die Leuchtdioden auch "proximal" angeordnet werden und ihr Licht in Faserbündel einkoppeln.

Durch die erfindungsgemäße Ausbildung ergibt sich ein so heller Beleuchtungsfleck, dass dieser sogar im Licht von Operationsleuchten sichtbar ist. Damit ist es möglich, auf eine getrennte optische Zieleinrichtung mit einer eigenen Beleuchtungsquelle, wie einem Laserpointer zu verzichten. Der durch die Überlagerung des Lichts der einzelnen Fasern entstehende helle Lichtfleck dient dann als Zielmarke auch bei heller Umgebungsbeleuchtung.

Als Kamera können die verschiedensten Kameras, beispielsweise elektronische oder herkömmliche StillKameras eingesetzt werden. Besonders bevorzugt ist es jedoch, wenn die Kamera eine Videokamera insbesondere mit einer Autofokuseinrichtung ist.

Halteeinrichtungen, wie Stirnbänder oder Kopfhauben sind aus einer Vielzahl von Anwendungen und insbesondere medizinischen Anwendungen bekannt. Ebenso sind geeignete Miniaturkameras einschließlich deren Steuer- und Ausleseeinheiten und Beleuchtungseinrichtungen bekannt. Auch sind ringförmige Leuchten erhältlich, die ringförmig um das Objektiv einer Kamera angeordnet werden können, und die das Bildfeld der Kamera auch dann ausleuchten, wenn die Kamera über ein Zoomobjektiv und/oder wechselbare Objektive verfügt.

Alternativ und bevorzugt ist es möglich, dass der von der Beleuchtungseinrichtung beleuchtete Fleck dem Bildfeld des jeweils eingesetzten Objektivs oder der eingestellten Brennweite des Zoomobjektivs angepasst wird.

Bei einer weiteren Realisierung der Erfindung weist zur Herstellung einer festen Ortsbeziehung zwischen der Kamera und wenigstens einer weiteren, an dem Stirnband oder der Kopfhaube gehaltenen Einheit die Halteeinrichtung ein starres Element auf, an dem die Kamera und die weitere Einheit befestigt sind.

Das starre Element kann an dem Stirnband bzw. der Kopfhaube befestigt sein. Alternativ kann das starre Element ein starrer Ring sein, der Bestandteil des Stirnbandes bzw. der Kopfhaube ist.

Da sich ein starrer Ring anders als ein flexibles Stirnband nicht an die Kopfform des jeweiligen Trägers anpassen kann, ist bevorzugt an der Innenseite des starren Rings wenigstens ein aufblasbares Kissen angebracht, das zur Anpassung an die Kopfform des jeweiligen Trägers dient.

Bei der weiteren Einheit kann es sich prinzipiell um die verschiedensten Einheiten, wie beispielsweise einen Bearbeitungslaser handeln. Bei einer bevorzugten Weiterbildung der Erfindung ist die weitere Einheit ebenfalls eine Videokamera. Damit sind Aufnahmen aus unterschiedlichen Perspektiven oder Stereoaufnahmen möglich.

Die Versorgungseinheiten für die Leuchte(n) und die Kameraeinheit sind bevorzugt getrennt von der Stirn-Einheit angeordnet und mit der erfindungsgemäßen Einheit über wenigstens ein Kabel verbunden. Das Kabel weist bevorzugt eine Vielzahl von Lichtleitfasern auf, die das Licht von wenigstens einer Lampe zu dem oder den Leuchtmitteln leiten.

Insbesondere kann das Kabel eine Vielzahl von Lichtleitfasern aufweisen, die das Licht von wenigstens einer Lichtquelle zu dem (den) Leuchtmittel(n) leiten.

Um die Störung des Trägers der Stirn-Einheit möglichst gering zuhalten, ist es besonders bevorzugt, wenn die Beleuchtungseinrichtung und die Kamera-Steuer- und Energieversorgungseinheit über ein einziges Kabel mit der Kamera und dem (den) Leuchtmittel(n) verbunden ist.

Ein besonders einfachen Aufbau eilt man, wenn die Beleuchtungseinrichtung und die Kamera-Steuer- und Energieversorgungseinheit als Module in einem einzigen Gehäuse angeordnet sind, und für das einige Kabel ein Kombinationsstecker vorgesehen ist, der eine Mehrzahl von Seriensteckern für die verschiedenen Funktionen, wie Verbindung von Lichtleitkabeln, Betrieb eines Lasermoduls bzw. eines Videomoduls etc. in einem Kombinationsstecker-Gehäuse vereinigt, so dass alle Serienstecker gemeinsam in entsprechende Buchsen an den jeweiligen Modulen steckbar sind.

Die erfindungsgemäße Einheit eignet sich nicht nur zur Dokumentation von Vorgängen, sondern auch zur "Online-Übertragung" zu anderen Arbeitsplätzen.

Um dem Träger der Stirn-Einheit eine Kommunikation mit Dritten zu ermöglichen, ist es weiterhin bevorzugt, dass ein Audiomodul vorgesehen ist, das eine insbesondere bidirektionale Kommunikation zwischen dem Träger der Einheit und einem entfernten "Arbeitsplatz" ermöglicht. Damit kann eine Person an dem anderen Arbeitsplatz dem Träger der Stirn-Einheit Anweisungen und/oder Hilfestellungen bei seiner jeweiligen Tätigkeit geben.

Hierzu ist es von besonderem Vorteil, wenn ein Videomodul vorgesehen ist, das eine Übertragung des aufgenommenen Bildes zu einem entfernten "Arbeitsplatz" ermöglicht.

In jedem Falle ist es bevorzugt, wenn die Übertragung der Audio- und/oder Videosignale drahtlos erfolgt. Eine besonders einfachen Übertragung der Videosignale ist dann möglich, wenn die Videosignale komprimiert beispielsweise gemäß MPEG IV übertragen werden.

Um die Belastung für den Träger der Stirn-Einheit möglichst gering zuhalten, ist es bevorzugt, wenn die Module getrennt von der Stirn-Einheit angeordnet sind.

Die Kommunikation zwischen dem Träger der Stirn-Einheit und Dritten wird weiter verbessert, wenn die Einheit ein Headup-Display aufweist. Alternativ oder zusätzlich ist es möglich, dass eine Bedienungsperson an dem entfernten Arbeitsplatz die optische Zieleinrichtung steuern und insbesondere auf ein von der Bedienungsperson erkanntes Ziel ausrichten kann. Die Bedienungsperson an den entfernten Arbeitsplatz kann damit über die Zieleinrichtung direkt den Träger der Stirn-Einheit führen.

Wie bereits ausgeführt, ist es von Vorteil, wenn die Module nicht an der Halteeinrichtung angeordnet sind. Um die Bewegungsfreiheit des Trägers der Stirn-Einheit nicht zu beeinträchtigen, ist es von besonderem Vorteil, wenn eine Steuer- und Energieversorgungseinheit vorgesehen ist, die tragbar ist. Die Steuer- und Energieversorgungseinheit kann dabei in einem Rucksack untergebracht sein.

Um ein langes Arbeiten zu ermöglichen, ist es von besonderem vorteil, die Steuer- und Energieversorgungseinheit eine Brennstoffzelle mit einem Flüssiggasspeicher aufweist.

Weiterhin kann ein Archivierungssystem, eine Bildverarbeitungseinheit und/oder eine Anzeigeeinheit vorgesehen sein.

Aufgrund der vorstehenden Beschreibung kann ein auf dem einschlägigen Gebiet tätiger Fachmann eine erfindungsgemäße Stirn-Leuchten/Kamera-Einheit jederzeit ausführen, sodass auf die Beschreibung eines Ausführungsbeispiels anhand von Zeichnungen verzichtet werden kann.

## Patentansprüche

1. Stirn-Leuchten/Kamera-Einheit insbesondere für medizinische Anwendungen mit
- einer insbesondere elektronischen Kamera, wie einer Videokamera,
- einer Beleuchtungseinrichtung mit wenigstens einem Leuchtmittel, aus dessen Lichtaustrittsfläche das Licht zur Beleuchtung des Bildfeldes der Kamera austritt, und
- einer Halteeinrichtung, die das wenigstens eine Leuchtmittel und die Kamera am Kopf einer Person hält,
**dadurch gekennzeichnet, dass** eine optische Zieleinrichtung vorgesehen ist, die wenigstens eine Marke in das von dem Leuchtmittel beleuchtete Bildfeld der Kamera projiziert.

2. Einheit nach Anspruch 1,
**dadurch gekennzeichnet, dass** die Zieleinrichtung die Marke in das Zentrum des von der Kamera aufgenommen Bildfeldes projiziert.

3. Einheit nach Anspruch 1,
**dadurch gekennzeichnet, dass** die optische Zieleinrichtung mehrere Marken projiziert.

4. Einheit nach Anspruch 3,
**dadurch gekennzeichnet, dass** die Marken die Begrenzung des Bildfeldes angeben.

5. Einheit nach Anspruch 3,
**dadurch gekennzeichnet, dass** sich die Marken in einem Punkt vereinigen, wenn der Ort, auf dem sie auftreffen, im Fokusbereich der Kamera liegt.

6. Einheit nach einem der Ansprüche 1 bis 5,
**dadurch gekennzeichnet, dass** die optische Zieleinrichtung wenigstens einen Laserpointer aufweist.

7. Einheit nach einem der Ansprüche 1 bis 6,
**dadurch gekennzeichnet, dass** die Marken im Bild der Kameraeinheit elektronisch oder optisch ausgeblendet sind.

8. Einheit nach einem der Ansprüche 1 bis 7,
**dadurch gekennzeichnet, dass** die Beleuchtungseinrichtung wenigstens eine Lichtleitfaser aufweist, in die das Beleuchtungslicht eingekoppelt wird, und deren Lichtaustrittsende zusammen mit einer Linse, die den austretenden Lichtkegel auf das Bildfeld der Kamera abbildet, die Lichtaustrittsfläche des Leuchtmittels bildet.

9. Einheit nach Anspruch 8,
**dadurch gekennzeichnet, dass** eine Vielzahl von Lichtleitfasern vorgesehen ist, und
dass jeder Lichtleitfaser eine Linse derart zugeordnet ist, dass sich die Lichtbündel der einzelnen Lichtleitfasern im Bildfeld der Kamera überlagern.

10. Einheit nach Anspruch 9,
**dadurch gekennzeichnet, dass** jede Lichtleitfaser einen Durchmesser von ca. 0,5 mm hat.

11. Einheit nach einem der Ansprüche 8 bis 10,
**dadurch gekennzeichnet, dass** der durch die Überlagerung des Lichts der einzelnen Fasern entstehende helle Lichtfleck als Zielmarke auch bei heller Umgebungsbeleuchtung dient.

12. Einheit nach einem der Ansprüche 8 bis 11,
**dadurch gekennzeichnet, dass** die Beleuchtungseinrichtung wenigstens eine Lampe und insbesondere eine Gasentladungslampe aufweist, deren Licht in die Lichtleitfaser(n) eingekoppelt wird.

13. Einheit nach einem der Ansprüche 1 bis 11,
**dadurch gekennzeichnet, dass** die Beleuchtungseinrichtung wenigstens eine Leuchtdiode aufweist.

14. Einheit nach Anspruch 13,
**dadurch gekennzeichnet, dass** die wenigstens eine Leuchtdiode distal angeordnet ist, und das (die) Leuchtmittel bildet.

15. Einheit nach Anspruch 13 oder 14,
**dadurch gekennzeichnet, dass** die Leuchtdiode(n) Weißlichtdiode(n) oder im UV- oder IR-Bereich strahlende Dioden sind.

16. Einheit nach einem der Ansprüche 1 bis 15,
**dadurch gekennzeichnet, dass** die Leuchtmittel oder die Lichtaustrittsfläche eines Leuchtmittels das Objektiv der Kamera umgeben.

17. Einheit nach einem der Ansprüche 1 bis 16,
**dadurch gekennzeichnet, dass** die Kamera eine Videokamera insbesondere mit einer Autofokuseinrichtung ist.

18. Einheit nach Anspruch 17,
**dadurch gekennzeichnet, dass** die Videokamera ein Zoomobjektiv und/oder wechselbare Objektive aufweist.

19. Einheit nach Anspruch 18,
**dadurch gekennzeichnet, dass** der von dem (den) Leuchtmittel(n) beleuchtete Fleck dem Bildfeld des jeweils eingesetzten Objektivs oder der eingestellten Brennweite des Zoomobjektivs angepasst wird.

20. Einheit nach einem der Ansprüche 1 bis 19,
**dadurch gekennzeichnet, dass** die Halteeinrichtung ein Stirnband oder eine Kopfhaube aufweist.

21. Stirn-Leuchten/Kamera-Einheit insbesondere für medizinische Anwendungen mit
- einer insbesondere elektronischen Kamera, wie einer Videokamera,
- einer Beleuchtungseinrichtung mit wenigstens einem Leuchtmittel, aus dessen Lichtaustrittsfläche das Licht zur Beleuchtung des Bildfeldes der Kamera austritt, und
- einer Halteeinrichtung, die das wenigstens eine Leuchtmittel und die Kamera am Kopf einer Person hält,
oder nach einem der Ansprüche 1 bis 20,
**dadurch gekennzeichnet, dass** zur Herstellung einer festen Ortsbeziehung zwischen der Kamera und wenigstens einer weiteren, an dem Stirnband oder der Kopfhaube gehaltenen Einheit die Halteeinrichtung ein starres Element aufweist, an dem die Kamera und die weitere Einheit befestigt sind.

22. Einheit nach Anspruch 21,
**dadurch gekennzeichnet, dass** das starre Element an dem Stirnband bzw. der Kopfhaube befestigt ist.

23. Einheit nach Anspruch 21,
**dadurch gekennzeichnet, dass** das starre Element ein starrer Ring ist, der Bestandteil des Stirnbandes bzw. der Kopfhaube ist.

24. Einheit nach Anspruch 23,
**dadurch gekennzeichnet, dass** an der Innenseite des starren Rings wenigstens ein aufblasbares Kissen angebracht ist, das zur Anpassung an die Kopfform des jeweiligen Trägers dient.

25. Einheit nach einem der Ansprüche 21 bis 24,
**dadurch gekennzeichnet, dass** die weitere Einheit ebenfalls eine Videokamera ist.

26. Einheit nach Anspruch 25,
**dadurch gekennzeichnet, dass** die beiden Videokameras eine Stereoaufnahme ermöglichen.

27. Einheit nach einem der Ansprüche 1 bis 26,
**dadurch gekennzeichnet**, das die Beleuchtungseinrichtung und eine Kamera-Steuer- und Energieversorgungseinheit getrennt von der Stirn-Einheit angeordnet und mit dem (den) Leuchtmittel(n) und der Kamera
über wenigstens ein Kabel verbunden sind.

28. Einheit nach Anspruch 27,
**dadurch gekennzeichnet, dass** das Kabel eine Vielzahl von Lichtleitfasern aufweist, die das Licht von wenigstens einer Lichtquelle zu dem (den) Leuchtmittel(n) leiten.

29. Einheit nach Anspruch 27 oder 28,
**dadurch gekennzeichnet, dass** die Beleuchtungseinrichtung und die Kamera-Steuer- und Energieversorgungseinheit über ein einziges Kabel mit der Kamera und dem (den) Leuchtmittel(n) verbunden ist.

30. Einheit nach Anspruch 29 oder nach dem Oberbegriff des Anspruchs 1,
**dadurch gekennzeichnet, dass** die Beleuchtungseinrichtung und die Kamera-Steuer- und Energieversorgungseinheit als Module in einem einzigen Gehäuse angeordnet sind, und
für das einige Kabel ein Kombinationsstecker vorgesehen ist, der eine Mehrzahl von Seriensteckern für die verschiedenen Funktionen, wie Verbindung von Lichtleitkabeln, Betrieb eines Lasermoduls bzw. eines Videomoduls etc. in einem Kombinationsstecker-Gehäuse vereinigt, so dass alle Serienstecker gemeinsam steckbar sind.

31. Einheit nach dem Oberbegriff des Anspruchs 1 oder nach einem der Ansprüche 1 bis 30,
**dadurch gekennzeichnet, dass** ein Audiomodul vorgesehen ist, das eine insbesondere bidirektionale Kommunikation zwischen dem Träger der Einheit und einem entfernten "Arbeitsplatz" ermöglicht.

32. Einheit nach dem Oberbegriff des Anspruchs 1 oder nach einem der Ansprüche 1 bis 30,
**dadurch gekennzeichnet, dass** ein Videomodul vorgesehen ist, das eine Übertragung des aufgenommenen Bildes zu einem entfernten "Arbeitsplatz" ermöglicht.

33. Einheit nach Anspruch 31 oder 32,
**dadurch gekennzeichnet, dass** die Übertragung der Audio- und/oder Videosignale drahtlos erfolgt.

34. Einheit nach Anspruch 33,
**dadurch gekennzeichnet, dass** die Videosignale komprimiert beispielsweise gemäß MPEG IV übertragen werden.

35. Einheit nach einem der Ansprüche 31 bis 34,
**dadurch gekennzeichnet, dass** die Module getrennt von der Stirn-Einheit angeordnet sind.

36. Einheit nach dem Oberbegriff des Anspruchs 1 oder nach einem der Ansprüche 1 bis 34,
**dadurch gekennzeichnet, dass** ein Modul vorgesehen ist, das eine bidirektionale Kommunikation zwischen der Einheit und einem "entfernten Arbeitsplatz" ermöglicht.

37. Einheit nach Anspruch 36,
**dadurch gekennzeichnet**, das eine Bedienungsperson an dem entfernten Arbeitsplatz Informationen an den Träger der Einheit übermitteln kann.

38. Einheit nach Anspruch 37,
**dadurch gekennzeichnet, dass** die Einheit hierzu ein Headup-Display aufweist.

39. Einheit nach Anspruch 37 oder 38,
**dadurch gekennzeichnet, dass** eine Bedienungsperson an dem entfernten Arbeitsplatz die optische Zieleinrichtung steuern und insbesondere auf ein von der Bedienungsperson erkanntes Ziel ausrichten kann.

40. Einheit nach dem Oberbegriff des Anspruchs 1 oder nach einem der Ansprüche 1 bis 30,
**dadurch gekennzeichnet, dass** eine Steuer- und Energieversorgungseinheit vorgesehen ist, die tragbar ist.

41. Einheit nach Anspruch 40,
**dadurch gekennzeichnet, dass** die Steuer- und Energieversorgungseinheit in einem Rucksack untergebracht ist.

42. Einheit nach Anspruch 40 oder 41,
**dadurch gekennzeichnet, dass** die Steuer- und Energieversorgungseinheit eine Brennstoffzelle mit einem Flüssiggasspeicher aufweist.

43. Einheit nach einem der Ansprüche 1 bis 42,
**dadurch gekennzeichnet, dass** ein Archivierungssystem vorgesehen ist.

## Claims

1. Headlamp/camera unit particularly for medical applications with
a - particularly electronic camera, such as a video camera,
a lighting installation with at least one illuminant, emitting through its light emitting area the light for illuminating the image field of the camera, and
a holding device to hold the at least one illuminant and the camera on the head of a person, and which is **characterized by** the provision made for an optical sighting device that projects a minimum of one marker onto the image field of the camera, which is illuminated by the illuminant.

2. Unit pursuant to Claim 1, **characterized by** the fact that the sighting device projects the marker into the centre of the image field filmed by the camera.

3. Unit pursuant to Claim 1, **characterized by** the fact that the optical sighting device projects several markers.

4. Unit pursuant to Claim 3, **characterized by** the fact that the markers demonstrate the limits of the image field.

5. Unit pursuant to Claim 3, **characterized by** the fact that the markers unite in a single point if the location where they converge is within the focus field of the camera.

6. Unit pursuant to one of the Claim, between 1 and 5 **characterized by** the fact that the optical sighting device carries at least one laser pointer.

7. Unit pursuant to the Claims between 1 and 6, **characterized by** the fact that the markers in the image of the camera unit are suppressed electronically or optically.

8. Unit pursuant to one of the Claim between 1 and 7, **characterized by** the fact that the illuminating device has at least one optical fibre into which the illuminating light is coupled and the light emitting end of which, together with a lens, projects the light cone emitted onto the image field of the camera, thus forming the light emitting area of the illuminant.

9. Unit pursuant to Claim 8, **characterized by** the fact that provision is made for large number of optical fibres and that lens is allocated to each optical fibre in such manner that the light beam of the individual optical fibres are superimposed on the image field of the camera.

10. Unit pursuant to Claim 9, **characterized by** the approximately 0.5 mm diameter of each optical fibre.

11. Unit pursuant to one of the Claims between 8 and 10, **characterized by** the fact that the bright patch of light formed by the superimposition of the light of the individual fibres serves as a target marker even if the ambient light is bright.

12. Unit pursuant to one of the Claims between 8 and 11, **characterized by** the fact that the lighting installation has at least one lamp and in particular, a gas discharging lamp, the light of which is coupled into the optical fibre(s).

13. Unit pursuant to the Claims between 1 and 11, **characterized by** the fact that the lighting installation has at least one light diode.

14. Unit pursuant to Claim 13, **characterized by** the fact that at least one light diode is positioned directly on the camera head and forms the illuminant(s).

15. Unit pursuant to Claim 13 or 14, **characterized by** the fact that the light diode(s) are white light diode(s) or diodes that radiate in the UV or IR range.

16. Unit pursuant to the Claims between 1 and 15, **characterized by** the fact that the illuminants or the light emitting area of an illuminant surround the lens of the camera.

17. Unit pursuant to the Claims between 1 and 16, **characterized by** the fact that the camera is a video camera, in particular with an autofocus device.

18. Unit pursuant to Claim 17, **characterized by** the fact that the video camera has a zoom lens and/or exchangeable lenses.

19. Unit pursuant to Claim 18, **characterized by** the fact that the patch lit up by the illuminant(s) is adjusted to the image field of the respective lens in use or the focal length set on the zoom lens.

20. Unit pursuant to the Claims between 1 and 19, **characterized by** the fact that the holding device has a headband or cap.

21. Headlight/camera unit in particular for medical applications in particular with an electronic camera, such as a video camera,
a lighting installation with a minimum of one illuminant from the light emitting area of which the light is emitted to illuminate the image field of the camera and a holding device that holds the minimum of one illuminant and the camera on the head of a person,
or, pursuant to the Claims between 1 and 20, **characterized by** the fact that in order to create a fixed physical relationship between the camera and at least one further unit positioned on the headband or cap, the holding device has a rigid element in which the camera and the further unit are attached.

22. Unit pursuant to Claim 21 **characterized by** the fact that the rigid element is attached to the headband or cap.

23. Unit pursuant to Claim 22 **characterized by** the fact that the rigid element is a rigid ring.

24. Unit pursuant to Claim 23 **characterized by** the fact that at least one inflatable pad is attached to the inner ring of the rigid ring for the purpose of adjustment to the shape of the head of the respective wearer.

25. Unit pursuant to the Claims between 2 and 24, **characterized by** the fact that the further unit is also a video camera.

26. Unit pursuant to Claim 25 **characterized by** the fact that the two video cameras make it possible to film in stereo.

27. Unit pursuant to one of the Claims between 1 and 26, **characterized by** the fact that the lighting installation and a camera control and energy supply unit are positioned separately from the head unit and are connected to the illuminant(s) by at least one cable.

28. Unit pursuant to Claim 27 **characterized by** the fact that the cable had multiple optical fibres that direct the light of at least one light source to the illuminant(s).

29. Unit pursuant to Claim 27 or 28 **characterized by** the fact that the lighting installation and the camera control and energy supply unit are connected to the camera and illuminant(s) by means of single cable.

30. Unit pursuant to Claim 29, or the collective term of Claim 1.
**characterized by** the fact that the lighting installation and the camera control and energy supply and are positioned as modules in a single housing and that provision is made for a multiple plug for the several cables, which unites a number of serial plugs for the different functions such as connecting light conducting cables, operating a laser module or a video module or a etc. in a multiple plug housing so that all serial plugs can be plugged in together.

31. Unit pursuant to the collective term of Claim 1 or to one of the claims between 1 and 30, **characterized by** the fact provision is made for an audio module that facilitates in particular two-way communication between the person wearing the unit and a remote "workplace".

32. Unit pursuant to Claim 1 or to one of the claims between 1 and 30, **characterized by** the fact that provision has been made for video module that facilitates the transmission of the filmed image to a remote "workplace".

33. Unit pursuant to Claim 31 or 32, **characterized by** the fact that the transmission of audio and/or signals takes place without wire.

34. Unit pursuant to Claim 33, **characterized by** the fact that the video signals are transmitted in compressed form, for instance in compliance with MPEG IV.

35. Unit pursuant to Claims between 31 and 34, **characterized by** the fact that the modules are positioned separate from the head unit.

36. Unit pursuant to collective term of Claim 1 or pursuant to one of the Claims between 1 and 34, **characterized by** the fact that provision is made for a module that facilitates two-way communication between the unit and a "remote workplace".

37. Unit pursuant to Claim 36, **characterized by** the fact that a person operating at a remote workplace can transmit information to the person wearing the unit.

38. Unit pursuant to Claim 37, **characterized by** the fact that the unit has a head-up display for this purpose.

39. Unit pursuant to Claim 37 or 38, **characterized by** the fact that a person operating it controls the optical sighting device at a remote workplace and, in particular directs it towards a target recognized by the person operating the device.

40. Unit pursuant to the collective form to Claim 1, **characterized by** the fact that provision is made for a control and energy supply unit that is portable.

41. Unit pursuant to Claim 28 **characterized by** the fact that the control and energy supply unit is kept in a rucksack.

42. Unit pursuant to Claim 40 or 41 **characterized by** the fact that the control and energy supply unit has a fuel cell with a liquid gas reservoir.

43. Unit pursuant to one of the claim between 1 and 42, **characterized by** the fact that provision is made for an archiving system.

## Revendications

1. Unité frontale à fonctions éclairage/caméra, destinée en particulier à des applications médicales, du type se composant de :
- une caméra, en particulier électronique, telle qu'une vidéo - caméra ;
- un système d'éclairage, comprenant au moins un moyen d'éclairage, de la surface d'émission de lumière de laquelle provient la lumière destinée à l'éclairage du champ d'image de la caméra ; et
- un système de maintien, qui supporte sur la tête d'une personne le moyen d'éclairage et la caméra,
**caractérisée en ce qu'**il est prévu un système de visée optique qui projette au moins une marque dans le champ d'image éclairé par le moyen d'éclairage de la caméra.

2. Unité selon la revendication 1, **caractérisée en ce que** la marque est projetée au centre du champ d'image pris par la caméra.

3. Unité selon la revendication 1, **caractérisé en ce que** le système de visée projette plusieurs marques.

4. Unité selon la revendication 3, **caractérisé en ce que** les marques définissent les limites du champ d'image.

5. Unité selon la revendication 3, **caractérisé en ce que** les marques sont réunies en un site quand l'emplacement sur lequel elles frappent se situe dans la région focale de la caméra.

6. Unité selon l'une quelconque des revendications 1 à 5, **caractérisée en ce que** le système de visée comporte au moins un siteur à laser.

7. Unité selon l'une quelconque des revendications 1 à 6, **caractérisée en ce que** les marques sont ressorties de l'image de l'unité de caméra par voie électronique ou optique.

8. Unité selon l'une quelconque des revendications 1 à 7, **caractérisée en ce que** le système d'éclairage comporte au moins une fibre optique conductrice de la lumière, dans laquelle la lumière d'éclairage est introduite et dont les extrémités de sortie de lumière forment, sur le champ d'image de la caméra, en même temps qu'avec une lentille qui recueille le cône de lumière sortante, la surface de sortie du moyen d'éclairage.

9. Unité selon la revendication 8, **caractérisée en ce qu'**il est prévu une pluralité de fibres optiques conductrices de la lumière et **en ce que**, à chaque fibre optique est associée une lentille, de manière telle que les faisceaux de lumière des fibres individuelles conductrices de lumière se superposent dans le champ d'image de la caméra.

10. Unité selon la revendication 9, **caractérisée en ce que** chaque fibre conductrice de lumière présente un diamètre d'environ 0,5 mm.

11. Unité selon l'une quelconque des revendications 8 à 10, **caractérisée en ce que** la tache de lumière claire résultant de la superposition de la lumière des fibres individuelles sert également de cible dans le cas d'un éclairage environnant clair.

12. Unité selon l'une quelconque des revendications 8 à 11, **caractérisée en ce que** le système d'éclairage comporte au moins une lampe, et en particulier une lampe à décharge dans un gaz, dont la lumière est introduite dans la (les) fibre(s) conductrice(s) de lumière.

13. Unité selon l'une quelconque des revendications 1 à 11, **caractérisée en ce que** le système d'éclairage comporte au moins une diode lumineuse.

14. Unité selon la revendication 13, **caractérisée en ce que** la diode lumineuse est placée distalement et constitue la (les) moyen(s) d'éclairage.

15. Unité selon l'une ou l'autre des revendications 13 ou 14, **caractérisée en ce que** la (les) diode(s) lumineuse(s) sont des iodes à lumière blanche ou des diodes émettrices dans les régions UV ou IR.

16. Unité selon l'une quelconque des revendications 1 à 15, **caractérisée en ce que** le moyen d'éclairage ou la surface émettrice de lumière d'un moyen d'éclairage entourent l'objectif de la caméra.

17. Unité selon l'une quelconque des revendications 1 à 16, **caractérisée en ce que** la caméra est une vidéo -caméra, en particulier équipée d'un système auto-focus.

18. Unité selon la revendication 17, **caractérisée en ce que** la vidéo -caméra comporte un objectif « zoom » et/ou un objectif interchangeable.

19. Unité selon la revendication 18, **caractérisé en ce que** la tache éclairée par le(s) moyen(s) d'éclairage est adaptée au champ d'image de chaque objectif mis en place ou à la distance focale réglée de l'objectif zoom.

20. Unité selon l'une quelconque des revendications 1 à 19, **caractérisée en ce que** le système de maintien comporte un bandeau frontal ou une calotte.

21. Unité frontale à fonctions éclairage/caméra, destinée en particulier à des applications médicales, du type se composant de :
- une caméra, en particulier électronique, telle qu'une vidéo-caméra ;
- un système d'éclairage, comportant au moins un moyen d'éclairage, de la surface de sortie de lumière duquel sort la lumière destinée à éclairer le champ d'image de la caméra, et
- un système de maintien, destiné à supporter sur la tête d'une personne le moyen d'éclairage et la caméra,
ou selon l'une quelconque des revendications 1 à 20, **caractérisée en ce que**, en vue de la création d'une correspondance spatiale fixe entre la caméra et au moins une autre unité maintenue sur le bandeau frontal ou la calotte, le système de maintien comporte un élément rigide auquel sont fixés la caméra et l'autre unité.

22. Unité selon la revendication 21, **caractérisée en ce que** l'élément rigide est fixé au bandeau frontal ou à la calotte.

23. Unité selon la revendication 21, **caractérisée en ce que** l'élément rigide est un collier rigide qui constitue le composant du bandeau frontal ou de la calotte.

24. Unité selon la revendication 23, **caractérisée en ce que**, sur la face interne du collier rigide est monté au moins un coussin gonflable, qui sert à l'adaptation à la forme de la tête de chaque porteur.

25. Unité selon l'une quelconque des revendications 21 à 24, **caractérisée en ce que** l'autre unité est également une vidéo -caméra.

26. Unité selon la revendication 25, **caractérisée en ce que** les deux vidéo-caméra rendent possible une prise de vue stéréoscopique.

27. Unité selon l'une quelconque des revendications 1 à 26, **caractérisée en ce que** le système d'éclairage et une unité destinée à l'alimentation en énergie et à la commande de la caméra sont disposées séparées de l'unité frontale et sont réunies par au moins un câble au(x) moyen(s) d'éclairage et à la caméra.

28. Unité selon la revendication 27, **caractérisée en ce que** le câble se compose d'une pluralité de fibres conductrices de lumière, qui conduisent la lumière depuis une source de lumière jusqu'au(x) moyen(s) d'éclairage.

29. Unité selon l'une ou l'autre des revendications 27 ou 28, **caractérisée en ce que** le système d'éclairage et l'unité destinée à l'alimentation en énergie et à la commande de la caméra est reliée par un câble unique à la caméra et au(x) moyen(s) d'éclairage.

30. Unité selon la revendication 29 ou selon le préambule de la revendication 1, **caractérisée en ce que** le système d'éclairage et l'unité destinée à l'alimentation en énergie et à la commande de la caméra sont disposées sous forme d'un module dans un boîtier unique, pour le câble unique étant prévue une prise combinée, qui réunit, dans un boîtier de branchement combiné une pluralité de prises pour les diverses fonctions telles que la liaison des câbles conducteurs de lumière, l'actionnement d'un module à laser ou d'un module vidéo, etc, de sorte que toutes les prises peuvent être enfoncées ensemble en série.

31. Unité selon le préambule de la revendication 1 ou selon l'une quelconque des revendications 1 à 30, **caractérisée en ce qu'**il est prévu un module audio, qui rend possible, en particulier, une communication bidirectionnelle entre le porteur de l'unité et un « site de travail » éloigné.

32. Unité selon le préambule de la revendication 1 ou selon l'une quelconque des revendications 1 à 30, **caractérisée en ce qu'**il est prévu un module vidéo, qui rend possible un transfert de l'image reçue à un " site de travail " éloigné.

33. Unité selon l'une ou l'autre des revendications 31 ou 32, **caractérisée en ce que** le transfert des signaux audio et/ou vidéo se fait sans fil.

34. Unité selon la revendication 33, **caractérisée en ce que** les signaux vidéo sont transférés à l'état comprimé, par exemple selon le procédé MPG IV.

35. Unité selon l'une quelconque des revendications 31 à 34, **caractérisée en ce que** les modules sont séparés de l'unité frontale.

36. Unité selon le préambule de la revendication 1 ou l'une quelconque des revendications 1 à 34, **caractérisée en ce qu'**il est prévu un module qui rend possible une communication bidirectionnelle entre l'unité et un " site de travail " éloigné.

37. Unité selon la revendication 36, **caractérisée en ce qu'**une personne de service au site de travail éloigné peut transmettre des informations au porteur de l'unité.

38. Unité selon la revendication 37, **caractérisée en ce que** l'unité comporte à cet effet un affichage tête-haute.

39. Unité selon l'une ou l'autre des revendications 37 ou 38, **caractérisée en ce qu'**une personne de service au site de travail éloigné peut commander le système de cible optique et en particulier le diriger sur une cible connue de la personne de service.

40. Unité selon le préambule de la revendication 1 ou l'une quelconque des revendications 1 à 30, **caractérisée en ce qu'**il est prévu une unité pour l'alimentation en énergie et la commande qui soit transportable.

41. Unité selon la revendication 40, **caractérisée en ce que** l'unité pour l'alimentation en énergie et la commande est logée dans un sac à dos.

42. Unité selon l'une ou l'autre des revendications 40 ou 41, **caractérisée en ce que** l'unité pour l'alimentation en énergie et la commande comporte une cellule à combustible avec un réservoir de liquide.

43. Unité selon l'une quelconque des revendications 1 à 42, **caractérisée en ce qu'**il est prévu un système d'archivage.
